# EUROPEAN PATENT SPECIFICATION

(11) **EP 2 043 684 B1**
(45) Date of publication and mention of the grant of the patent: **01.11.2017**
(21) Application number: 07730314.7
(22) Date of filing: 28.06.2007
(51) Int. Cl.: A61K 39/35, A61K 39/36

(54) **A METHOD FOR THE PRODUCTION OF HYDROLYZED ALLERGEN**
VERFAHREN ZUR HERSTELLUNG VON HYDROLYSIERTEM ALLERGEN
PROCÉDÉ DE PRODUCTION D'UN ALLERGÈNE HYDROLYSÉ

(30) Priority: 29.06.2006 EP 06116322; 06.09.2006 US 842485 P
(43) Date of publication of application: 08.04.2009
(73) Proprietor: ASIT BioTech s.a., 1200 Bruxelles (BE)
(72) Inventor: LEGON, Thierry, B-3360 Bierbeek (BE); PIROTTON, Sabine, B-1070 Brüssel (BE); PLACIER, Gael, B-1030 Brussels (BE); KERGOAT, Gilles, B-1030 Brussels (BE)
(74) Representative: dompatent von Kreisler Selting Werner - Partnerschaft von Patent- und Rechtsanwälten mbB
(86) International application number: PCT/EP2007/056454
(87) International publication number: WO 2008/000783

(56) References cited:
- WO-A-92/19970
- WO-A-99/22762
- WO-A-2004/112833
- DE-A1- 19 939 982
- US-A1- 2004 071 738
- US-A1- 2006 110 435
- ISHIZAKA K ET AL: "IMMUNOGENIC PROPERTIES OF MODIFIED ANTIGEN E PART 2 ABILITY OF UREA DENATURED ANTIGEN AND ALPHA POLY PEPTIDE CHAIN TO PRIME THYMUS DERIVED CELLS SPECIFIC FOR ANTIGEN E" JOURNAL OF IMMUNOLOGY, vol. 114, no. (1 PART 1), 1975, pages 110-115, XP009076696 ISSN: 0022-1767
- ZHU XIAOJIU ET AL: "T cell epitope mapping of ragweed pollen allergen Ambrosia artemisiifolia (Amb a 5) and Ambrosia trifida (Amb t 5) and the role of free sulfhydryl groups in T cell recognition", JOURNAL OF IMMUNOLOGY, AMERICAN ASSOCIATION OF IMMUNOLOGISTS, US, vol. 155, no. 10, 1 January 1995 (1995-01-01), pages 5064-5073, XP002509111, ISSN: 0022-1767
- DATABASE BIOSIS [Online] BIOSCIENCES INFORMATION SERVICE, PHILADELPHIA, PA, US 1983 SUGANE K ET AL: 'PURIFICATION AND CHARACTERIZATION OF EXCRETORY AND SECRETORY ANTIGEN OF TOXOCARA-CANIS LARVAE' Database accession no. PREV198477035559 & SUGANE K ET AL: "PURIFICATION AND CHARACTERIZATION OF EXCRETORY AND SECRETORY ANTIGEN OF TOXOCARA-CANIS LARVAE", IMMUNOLOGY, vol. 50, no. 1, 1983, pages 113-120, XP009159369, ISSN: 0019-2805
- JUTEL M ET AL: "Allergen-specific immunotherapy with recombinant grass pollen allergens", JOURNAL OF ALLERGY AND CLINICAL IMMUNOLOGY, ELSEVIER, AMSTERDAM, NL, vol. 116, no. 3, 1 September 2005 (2005-09-01), pages 608-613, XP026968490, ISSN: 0091-6749 [retrieved on 2005-09-11]
- LINHART BIRGIT ET AL: "Combination vaccines for the treatment of grass pollen allergy consisting of genetically engineered hybrid molecules with increased immunogenicity", FASEB JOURNAL, FED. OF AMERICAN SOC. FOR EXPERIMENTAL BIOLOGY, US, vol. 16, no. 10, 1 August 2002 (2002-08-01), pages 1301-1303, XP002583819, ISSN: 0892-6638
- VALENTA R ET AL: "REVIEW: The recombinant allergen-based concept of component-resolved diagnostics and immunotherapy /CRD and CRIT)", CLINICAL & EXPERIMENTAL ALLERGY : JOURNAL OF THE BRITISH SOCIETY FOR ALLERGY AND CLINICAL IMMUNOLOGY, WILEY INTERSCIENCE, UK, vol. 29, 1 January 1999 (1999-01-01), pages 896-904, XP002974433, ISSN: 0954-7894, DOI: 10.1046/J.1365-2222.1999.00653.X
- P. Moingeon: "Sublingual immunotherapy: from biological extracts to recombinant allergens", Allergy, vol. 61, no. s81, 1 July 2006 (2006-07-01) , pages 15-19, XP055097868, ISSN: 0105-4538, DOI: 10.1111/j.1398-9995.2006.01157.x

## Description

The present invention is related to a new method for the production of a purified allergen hydrolysate and the allergen hydrolysate obtainable by the new method.

Common allergens are pollens, house dust mites, moulds, drugs, foods and animal hair and dander.

The most common allergy diseases are rhinitis, asthma and atopic dermatitis. Allergic asthma is a chronic inflammatory disorder. Symptomatic treatment of allergic disorders is effected by use of antihistaminics, β-antogonists and corticosteroids.

Furthermore, the so called "specific" immunotherapy is based on a hyposensitization. Typically patients are administered with subcutaneous injection of the specific offending allergens. Treatment is started with small allergen doses and the doses are increased. Treatment is typically maintained for several years. This type of treatment suffers from pure patient compliance and has been questioned due to safety reasons because a patient can suffer from severe anaphylactic reactions.

In addition to methods comprising repeated subcutaneous injections there are also oral hyposensitization methods.

US 4,822,611 discloses a method for treating allergies comprising oral treatment with allergens. It describes the use of commercially available "bulk" allergenic extracts showing batch-to-batch variation and differences in extracts from different manufactures. The preparation of these extracts is not described.

GB 1 247 614 discloses a method of extracting an allergen. The aim of this method is to have a more complete and effective allergenic extract by including all extractable components of the allergen.

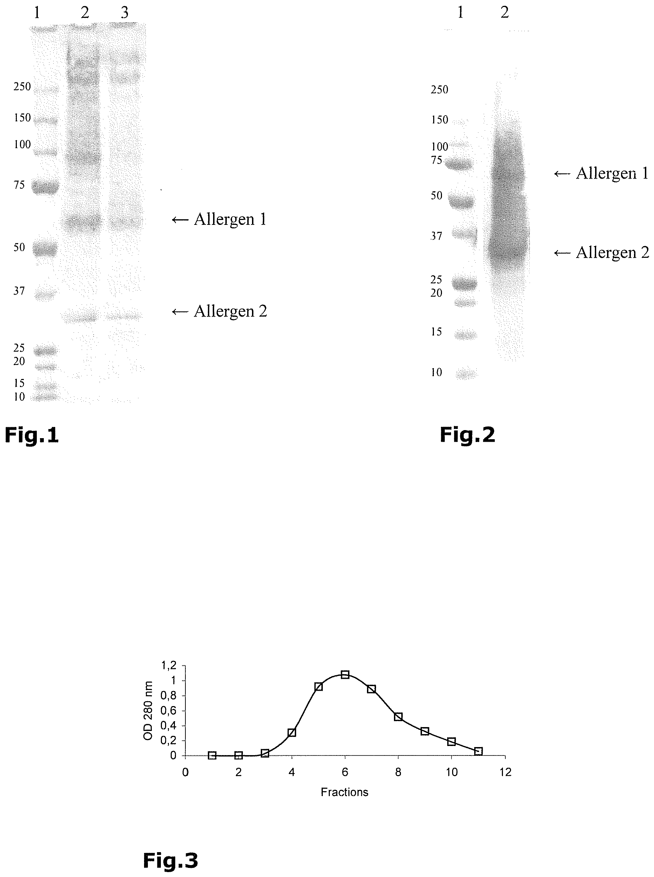

US 5,770,698 discloses a process for purifying extracts of allergenically active proteins. The spectrum of figure 2 does not present a peak at 280ₙₘ. This implies that the extract contains significant amount on non-protein impurities. WO 99/22762 discloses a similar method, therefore, the product comprises large amounts of non-protein impurities, too.

On the other hand, there has been a tendency to develop highly specific preparations based on single epitopes. For example, WO 00/58349 discloses an isolated and purified peptide comprising a leucin positioned two peptide bonds away from a tyrosine/arginine pair. These peptides can be used to prepare a pharmaceutical composition to accomplish treatment or prophylaxis, in this case especially directed to canine allergy in dogs.

On the one hand, methods are used to purify a specifically identified single allergenic molecule. On the other hand, people are trying to produce allergenic extracts as complete as possible.

According to the first alternative, it is always possible that the allergen preparation lacks the relevant epitopes to induce tolerance in a determined patient. The second alternative has a drawback of batch-to-batch variability and of the presence of compounds able to trigger immune response like DNA molecules, carbohydrates, lipids of complexes thereof.

It is an object of the present invention to overcome at least some of the drawbacks of prior art, especially to provide antigens from natural allergens with a significant reduced capability to trigger allergenicity reaction compared to the crude allergen extract but able to stimulate T-cells as well.

The problem is solved by the provision of methods for preparing an allergen extract comprising most of the protein containing parts of an allergen extract but with a reduced, preferably very low content of non-protein components such as nucleic acids, lipids, sugars and the like.

The extracts prepared according to the invention are superior to extracts of prior art, especially do they show a reproducible composition of proteins but are not purified to a single epitope.

A method for the production of a purified allergen hydrolysate comprising the steps of
a) extracting a natural source of allergens comprising allergenic proteins to form an extract,
b) purifying of said extract to remove non-protein components to form a purified extract,
c) denaturating said purified extract to form a purified denaturated extract, wherein denaturation is performed with mixtures of chaotropic agents and reducing agents,
   said purified denaturated extract comprising proteins, wherein no single protein is 60% or more (w/w) of all proteins measured by SDS-PAGE followed by densitometry and all proteins represent at least 60% (w/w) of the dry weight of the purified denaturated extract measured by BCA assay,
d) hydrolysing said purified denaturated extract with an enzyme to form an allergen hydrolysate,
e) purifying said allergen hydrolysate to remove peptides with a molecular weight above 10,000 Da and below 1,000 Da in order to obtain a purified hydrolysate where 70%, more preferably 80% of the peptides are between 10,000 Da and 1,000 Da.

Steps a) to c) referred to as method part I.

In contrast to the methods of prior art, the method of the present invention produces allergen extracts which comprise predominantly proteins without purifying the extract to a single peptide or protein.

In contrast to the products of prior art the products of the invention have following advantages:
- Immunogenic substances other than proteins are substantially removed
- The natural allergen extract is able to stimulate T-cell with the reduced ability to trigger immediate allergic reaction (basophile activation, mast cell degranulation)

As starting materials different natural occurring allergens can be used. Typical natural starting materials are milk, venom, egg, weed, grass, tree, shrub, flower, vegetable, grain, fungi, fruit, berry, nut, seed, bean, fish, shellfish, seafood, meat, spices, insect, mite, mould, animal, pigeon tick, worm, soft coral, animal dander, nematode, Hevea brasiliensis, and mixtures thereof.

After extraction of the material, the extract is purified to remove non-protein components such as sugars, lipids, nucleic acids and the like. Typical, several different proteins are present in the protein fraction of the purified extract.

According to prior art, one protein is purified and the other remaining proteins are "impurities".

In contrast thereto, it is the aim of the present invention to purify the proteins together. The relative amounts of the proteins in the purified extract can be easily measured using methods like SDS-PAGE followed by densitometry.

For 60% of total weight of the proteins, it is necessary to combine the two most dominant proteins at least, i.e. no single protein is 60% (w/w) or more of all proteins. More preferably, 60% of all proteins are formed by the at least 3 dominant proteins, preferably by the at least 4 dominant proteins and more preferably by at least 5, 6, 7, 8, 9 or 10 proteins.

For example, there are the following proteins:

| | |
|---|---|
| Protein 1: | 27% |
| Protein 2: | 13% |
| Protein 3: | 34% |
| Protein 4: | 19% |
| Protein 5: | 17% |

The most dominant proteins forming together at least 60% (≈ 60% or more) are proteins 3+1 (34+27=61%).

Furthermore, the total protein content of the purified extract is at least 60% by weight, preferably the content is at least 70% by weight or 80% by weight, more preferably 90% by weight of the purified extract.

Extraction is preferably performed with aqueous solutions. Suitable salts are salts such as but not restricted to carbonate, bicarbonate, phosphate, acetate, TRIS and HEPES.

Also in contrast to many other extraction methods, it is preferred that the amount of extraction medium is comparatively large, i.e. at least 20 times the weight of the natural source of allergens, preferably 100 time the weight or more.

Purifying of the extract may be performed by one or more of the following:
- ion exchange chromatography steps (including anion exchange chromatography and cation exchange chromatography),
- size exclusion chromatography step (also called gel filtration),
- precipitation steps,
- hydrophobic interaction chromatography steps,
- pseudo-affinity and affinity chromatographies and/or
- diafiltration.

In a preferred embodiment ion exchange chromatography is used wherein in case of a cation exchanger the loading solution has a pH between the pKa of the acidic function of the cation exchanger and the pKa of the protein having the lowest pKa of the proteins in the extract. In case of an anion exchanger the pH is between the pKa of the basic function of the anion exchanger and the pKa of the protein having the highest pKa of the proteins constituting the extract.

Through this method all proteins bind to the ion exchanger while the neutral impurities and the impurities with the same charge as the ion exchange resin will be removed.

In a preferred embodiment, at least one purification step is performed with a solution comprising one or more of a tenside and/or a denaturating agent. The tenside may be non-ionic, anionic, cationic or amphoteric. Suitable denaturating agents are chaotropic agents, reducing agents and mixtures thereof. Suitable denaturating agents are for example urea, guanidinium chloride, ethylene glycol, isopropanol. A suitable concentration of urea is 3 M or more, preferably 4 M or more. A suitable concentration of guanidinium is preferably 2 M, preferably 3 M or more. A suitable concentration of ethylene glycol and/or isopropanol is 5% or more, more preferably 10% or more, up to 20% by weight.

In some cases, the production of the purified extract according to the method part I of the disclosure is sufficient. Extracts of this type may be used to produce *ex vivo* / *in vivo* and *in vitro* diagnosis, prophylactic and therapeutic treatment of allergic diseases. A further aspect of the present disclosure is a method for the production of an allergen hydrolysate, either from extracts according to method part I or from any other source. If the extract comes from any other source of purified allergens than method part I, one preliminary step of denaturation is required in order to improve digestibility.

Steps d) and e) are referred to as method part II.

The advantages of the product obtained thereby are that the peptides are the digestion result of denaturated proteins. Due to a specified size calibration they have a reduced potency to induce immediate allergic reaction and pro-inflammatory reaction as well.

Denaturating, if necessary is preferably performed in the presence of chaotropic agents, reducing agents or mixtures thereof. Suitable chaotropic agents are for example urea and guanidinium chloride. Typical reducing agents are for example dithiotriethol, β-mercaptoethanol, thio-glycerol and mixtures thereof.

The hydrolysing step is typically performed with an enzyme. Suitable enzymes are for example pepsin, trypsin, chymotrypsin. This hydrolyzing step can be performed in the presence of a chaotropic agent, preferably urea or guanidinium chloride, too. During hydrolysing the concentration of urea and guanidinium chloride should be below 4 M, preferably below 3M.

In step e) of the method, peptides with a molecular weight larger than 10,000 Da or smaller than 1,000 Da, are removed.

The peptides of the purified hydrolysate, therefore, comprise peptides with a molecular weights between 1,000 and 10,000 Da. Suitable methods for removing large or small peptides are ultrafiltration and size exclusion chromatography. Again this size exclusion chromatography may be performed in the presence of a chaotropic agents, for example urea, guanidinium chloride, ethylene glycol, isopropanol and mixtures thereof.

A further disclosure is an allergen extract obtainable by method part I. Typically also in this extract the most dominant proteins by weight, which form together at least 60% by weight of all the proteins, are at least 2 proteins, preferably at least 3 or 4 proteins or more preferred at least 5, 6, 7, 8, 9 or 10 proteins. The purity is seen by a Optical Density 260ₙₘ: Optical Density 280ₙₘ-ratio of < 1, preferably < 0.9, more preferably between 0.75 and 0.9.

The allergen hydrolysate can be used for
- *in vivo* diagnosis of allergic diseases: prick tests, intracutaneous injections, conjonctival, sniff and inhalation tests
- *ex vivo* and *in vitro* diagnosis of allergic diseases: ELISA kits or standards to be used in tests
- Prophylatic and therapeutic treatments of allergic diseases: vaccine for de-sensitization/hyposensitization treatments and modulation of immune response with/without adjuvant combination.

A further embodiment of the present invention is a pharmaceutical composition comprising the allergen extract of the present invention in hydrolyzed form. Additionally, pharmaceutical composition may comprise one or more of the following substances: nucleoside triphosphates, nucleoside diphosphates, nucleoside monophosphates, nucleic acids, peptide nucleic acids, nucleosides or analogs thereof, immunosuppressive cytokines, compounds inducing expression of immunoproteasomes, 1,25-dihydroxyvitamin D3 or analogs thereof, lipopolysaccharides, endotoxins, heat shock proteins, thioredoxin with either NADPH or NADP-thioredoxin reductase, dithiothreitol, adrenergic receptor agonists such as salbutanol, adrenergic receptor antagonists such as butoxamine, compounds that regulate the expression of the adhesion molecule ICAM-1, N-acetyl-L-cysteine, y-L-glutamyl-L-cysteinyl-glycine (reduced L-glutathione), alpha-2-macroglobulins, inducers for Foxp3 gene expression, flavonoids, isoflavonoids, pterocarpanoids, stilbenes such as resveratrol, tachykinin receptor antagonists, chymase inhibitors, vaccine adjuvant like CpG or MPL or tolerogenic adjuvant like zymosan, beta-1,3-glucan, regulatory T-cell inducer, a muco-adhesive agent for attaching the particle to the intestinal mucosal lining such as a plant lectin, zinc, zinc salts, polysaccharides, vitamins and bacterial lysates.

Based on the source of natural allergens in the composition may comprise allergens selected among pollen allergens, milk allergens, venom allergens, egg allergens, weed allergens, grass allergens, tree allergens, shrub allergens, flower allergens, vegetable allergens, grain allergens, fungi allergens, fruit allergens, berry allergens, nut allergens, seed allergens, bean allergens, fish allergens, shellfish allergens, seafood allergens, meat allergens, spices allergens, insect allergens, mite allergens, mould allergens, animal allergens, pigeon tick allergens, worm allergens, soft coral allergens, animal dander allergens, nematode allergens, allergens of Hevea brasiliensis.

In a preferred embodiment, the pharmaceutical composition is prepared for oral administration, for sublingual drug delivery, for enteric drug delivery.
Figure 1: Immunoreactivity by IgG western-blot. Lane1 : molecular weight markers, lane 2 : crude protein extract, lane 3 : purified allergen denaturated extract. Membrane blocked by BSA 5 % and milk 3%. Patient serum diluted to 1/250. IgG binding was detected by goat anti-human IgG HRP diluted to 1/2,500 and revealed by TMB substrate. Allergen 1 : ± 61-54 kDa, Allergen 2: ± 36-31 kDa.
Figure 2: Immunoreactivity by IgE western-blot. Lane1 : molecular weight markers, lane 2 : crude protein extract, lane 3 : purified proteins. Membrane blocked by BSA 5 % and milk 3%. Patient serum diluted to 1/5. IgE binding detected by goat anti-human IgE HRP diluted to 1/10,000 and revealed by TMB substrate. Allergen 1 : ± 61-54 kDa Allergen 2: ± 36-31 kDa.
Figure 3: Exclusion peak of SEC G25 elution profile. The ratio column volume / sample volume was 12. The resin was equilibrated with Tris.HCl 25 mM, urea 1.5 M, pH 8.0 at a flow rate of 9 ml/min. The elution was followed by the absorbace at 280 nm.

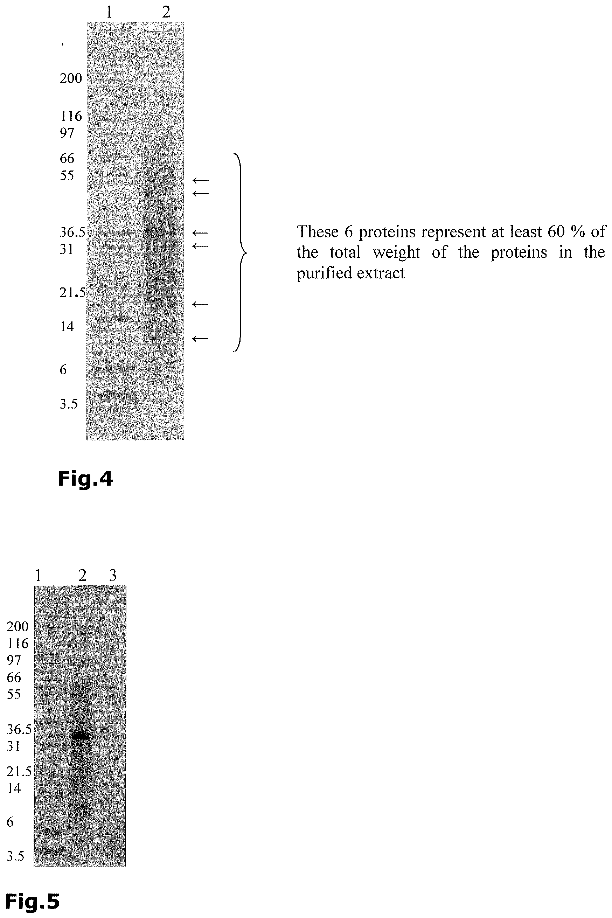

Figure 4: Protein profile by SDS-PAGE. 4 - 12% Bis-Tris gel. Lane 1 : molecular weight markers, lane 2 : purified allergen denaturated extract. Staining performed with Coomassie brillant blue R-250.
Figure 5: Protein and peptide profiles by SDS-PAGE. 4 - 12% Bis-Tris gel. Lane 1 : molecular weight markers, lane 2 : purified allergen denaturated extract (13 µg), lane 3 : hydrolysate (13 µg). Staining performed with Coomassie brillant blue R-250.

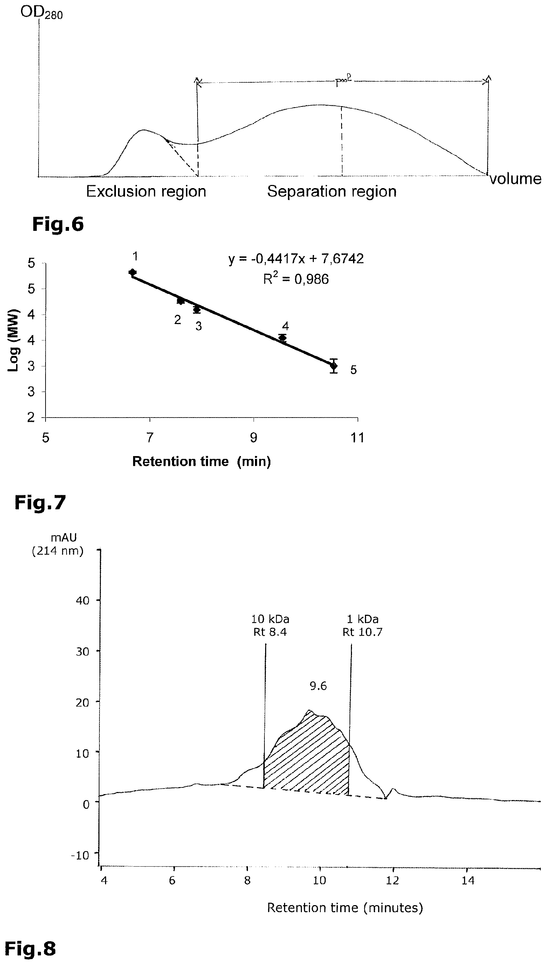

Figure 6: G50 SEC elution profile. The column was equilibrated with urea 2 M, NaCl 100 mM, pH 3.0. Flow rate 15 ml/min. The ratio column volume / sample volume was 10. The elution was followed by the absorbance at 280 nm.
Figure 7: Calibration curve for HPLC analysis. 10 µl of the following standards (1 mg/ml) were injected onto the BioSep-SEC S2000 column: 1. Bovine Serum Albumin (66 kDa), 2. β-Lactoglobulin (18.5 kDa), 3. Cytochrome C (12 kDa), 4. Glucagon (3.5 kDa), 5. 1 kDa synthetic peptide.
Figure 8: Size exclusion HPLC profile. Column : BioSep-SEC S2000 (PHENOMENEX). Elution buffer : Na₂HPO₄ 50 mM - SDS 0.5% (w/v) pH 6.8. Flow rate 1 ml/min. Detection at 214 nm. 10 µl of the samples were injected. The area under the curve, between 10 kDa and 1 kDa limits was used to calculate the percentage of the peptides of interest.

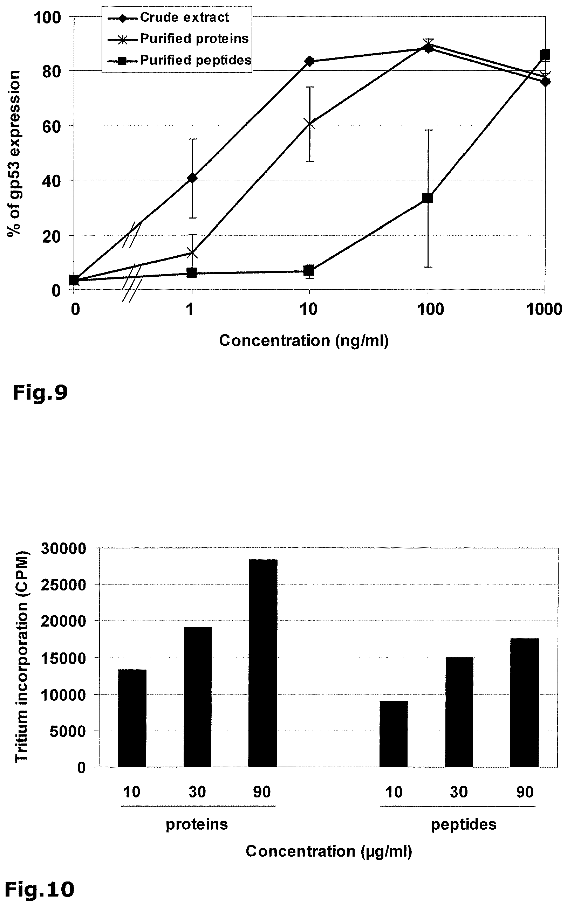

Figure 9: Allergenicity properties of the pollen-derived products. Blood samples from pollen allergic volunteers were incubated with increasing concentrations (0, 1, 10, 100 and 1000 ng/ml) of either pollen crude extract, pollen purified proteins and pollen purified peptides. gp53 protein expression was measured by flow cytometry with gating on IgE-positive leukocytes. Results are expressed as % of gp53 positive cells in activated cells (mean ± deviation of 2 determinations).
Figure 10: Stimulation of human PBMC proliferation by pollen proteins and pollen peptides. Human PBMC purified from pollen allergic volunteers were incubated 5 days at 37°C in the presence of increasing concentrations (10 30 and 90 µg/ml) of pollen proteins or pollen peptides. [³H]-Thymidine was added to the cell culture for 16 hours and the incorporation of [³H]-Thymidine was measured with a beta counter using the principle of liquid scintillation. Results are expressed as mean of 5 determinations. The method of the present invention is further exemplified by the following, non-limiting examples.

### Examples

### Example 1: Extraction

1% (w/v) pollen (*Lolium perenne* from ALLERGON) was added to sodium bicarbonate (12.5 mM) and incubated 2 h under stirring. The solution was then clarified and filtrated by adding celite (ACROS) at 2% (w/v) and passing through a 0.2 µm filter. This sample constitutes the crude extract.

The presence of allergens in the extract was analyzed by western blotting using pollen allergic patient sera. IgG and IgE epitopes are visualised with anti-human IgG or IgE antibodies.

As shown on figure 1 and 2, there are two major allergens in the extract.

The said crude extract was acidified to pH 3.0 and Tween 20 (0.1%, v/v) was added. This sample constitutes the acidified extract.

### Example 2: Purification of allergen proteins

The allergen extract was purified by:
- Cation exchange chromatography
   A sartobind S⁻ membrane (SARTORIUS) was equilibrated with 28x Bed volume (Bv) of sodium bicarbonate 12.5 mM, citrate 30 mM, pH 3.0, Tween 20 0.1% (v/v). The said acidified extract was loaded on the equilibrated membrane. The column was washed first with 35x Bv of sodium bicarbonate 12.5 mM, citrate 30 mM, pH 3.0, Tween 20 0.1% (v/v) and then washed with 42x Bv of sodium bicarbonate 12.5 mM, citrate 30 mM, pH 3.0. The proteins were eluted with carbonate 0.1 M, sodium chloride 0.5 M, pH 9.15. The presence of proteins was followed the OD at 280 nm. The fractions of interest were pooled.
- Ammonium sulfate precipitation
   This step was performed at 0-4°C.
   A quantity of ammonium sulfate to reach 90% of saturation was added to the product under stirring. The stirring was stopped after the complete dissolution of the salt. The suspension was incubated overnight and centrifuged 2 times during 15 min at 10,000 g. The supernatant was each time carefully discarded.
- Denaturation
   The pellets were resuspended at 9 mg/ml in urea 6 M, DTT 10 mM, Tris.HCl 0.1 M, pH 8.0 and incubated at 37°C for 1 h.
- Size exclusion chromatography on G25 resin (fine Sephadex from AMERSHAM)
   The denatured sample was loaded on the column and the proteins were eluted with Tris.HCl 25 mM, urea 1.5 M, pH 8.0.

The presence of proteins was followed by the OD measurement at 280 nm The fractions of interest were pooled to constitute the purifed denaturated allergen extract.

The purified allergen extract was further analysed. The protein content (BCA Assay) and the dry weight were determined in order to evaluate the protein purity. The purification efficiency was also followed by the removal of carbohydrates (Orcinol test) and by the decrease of the ratio OD₂₆₀ / OD₂₈₀.

**Table 1: Removal of non-protein components to form a purified extract**

| | Ratio protein / dry weight | Ratio OD₂₆₀/OD₂₈₀ | Ratio carbohydrates / proteins |
|---|---|---|---|
| Crude extract | 16% | 1.3 | 400% |
| Purified extract | 85% | 0.75 | 17% |

As shown in table 1, the purification process allows
- The increase of the percentage of proteins in the extract from ∼ 15% to 80%
- The OD₂₆₀/OD₂₈₀ ratio to tends towards 0.5 characterizing a pure protein
- A significant removal of carbohydrates (the residual content could represent the carbohydrate moiety of the proteins).

Figure 4 illustrates a typical SDS-PAGE profile obtained for the purified denaturated allergen extract. As can be seen, 6 proteins represent at least 60% of the total weight of the proteins in the purified extract.

### Example 3: Hydrolysis of denatured allergen extract

The extract was hydrolyzed using the following protocol :
The said purified allergen extract was acidified to pH 2.0. The digestion was performed at 2.5 mg/ml of pollen proteins and 1 Eu. Ph. U of pepsin (MERCK) for 337 mg of proteins, at 37°C, during 2 h.

Figure 5 shows a comparison between the purified extract (lane 2) and the hydrolyzed extract (lane 3). As can be seen, high molecular weight proteins corresponding to denatured undigested proteins have disappeared after the incubation with pepsin.

### Example 4: Purification

In order to eliminate the peptides with a MW ≥ 10,000 Da and MW ≤ 1,000 Da, the hydrolysate was purified by
- Size exclusion chromatography on G50 resin (fine Sephadex from AMERSHAM) 16.5 % (v/v) of isopropanol and 0.1 M of NaCl were added to the hydrolysate. This sample was immediately loaded on a G50 column. The peptides were eluted and the fractions containing the peptides (MW ≤ 10 kDa) were pooled as shown in figure 6.
- Diafiltration on 1kda membrane (ultrafiltation cassette Omega PES from PALL) The peptides were concentrated 10 x, diafiltrated against 10 volumes of Tris.HCl 50 mM pH 7.4 and finally concentrated 2.5 x. This sample constitutes the purified allergen hydrolysate.

The efficiency of the purification was controlled by size exclusion HPLC. A BioSep-SEC S2000 column (PHENOMENEX) was equilibrated with Na₂HPO₄ 50 mM - SDS 0.5% (w/v) pH 6.8 at a flow rate of 1 ml/min. The peptides were detected at 214 nm.

The 10 kDa and 1 kDa limits were calculated from a calibration curve as exemplified in figure 7.

As shown on figure 8, peptides with a molecular weight between 1,000 Da and 10,000 Da represent about 75% of all peptides in the purified hydrolysate.

### Example 5: Decrease of allergenicity

Allergenicity properties of the pollen crude extract (according to example 1), purified pollen proteins (according to example 2) and purified pollen peptides (according to example 4) were assessed by measuring their capacity to induce basophile degranulation.

The test was performed *in vitro* on fresh human blood samples from pollen allergic volunteers incubated with increasing concentrations of pollen crude extract, purified proteins and purified peptides. Basophile degranulation was assessed by measuring, by flow cytometric method, the expression of the gp53 protein marker on the cell membrane of activated cells (*i.e*. IgE positive cells). This protein is normally present within the membrane of the granules in resting cells and appears on the cell surface upon cell activation (due to the fusion of the granule membrane with the cytoplasmic membrane). It therefore becomes detectable by labeled specific anti-gp53 antibodies. As shown on figure 9, purified peptides are about 30x less allergenic than purified proteins and 100x less allergenic than pollen crude extract.

### Example 6 : Immunogenicity of the pollen proteins and pollen peptides

The immunogenicity of the allergen proteins and peptides was studied by measuring their ability to stimulate human peripheral blood mononuclear cell (PBMC) proliferation.

PBMC purified from blood sample from "pollen-allergic" volunteers by density gradient centrifugation were cultured 5 days in 96-well plates in the presence of increasing concentrations of pollen proteins and pollen peptides. On day 5, [³H]-Thymidine was added to the cell culture and the plates were further incubated at 37°C for 16 hours. Incorporation of [³H]-Thymidine was measured with a beta counter using the principle of liquid scintillation.

Pollen proteins (according to example 2) and pollen peptides (according to example 4) stimulate the proliferation of human PBMC in a dose concentration dependant way. Proliferation induced by allergen peptides is slightly lower than that observed in response to proteins. These results show that the process of peptide production conserves most of the epitopes of the allergen implicated in T cell activation.

## Claims

1. A method for the production of a purified allergen hydrolysate comprising the steps of
a) extracting a natural source of allergens comprising allergenic proteins to form an extract,
b) purifying of said extract to remove non-protein components to form a purified extract,
c) denaturating said purified extract to form a purified denaturated extract, wherein denaturation is performed with mixtures of chaotropic agents and reducing agents,
said purified denaturated extract comprising proteins, wherein no single protein is 60% or more (w/w) of all proteins measured by SDS-PAGE followed by densitometry and all proteins represent at least 60% (w/w) of the dry weight of the purified denaturated extract measured by BCA assay,
d) hydrolysing said purified denaturated extract with an enzyme to form an allergen hydrolysate,
e) purifying said allergen hydrolysate to remove peptides with a molecular weight above 10,000 Da and below 1,000 Da in order to obtain a purified hydrolysate where 70%, more preferably 80% of the peptides are between 10,000 Da and 1,000 Da.

2. The method of claim 1 wherein extracting is performed in a solution comprising no salt or a salt selected from carbonate, bicarbonate, phosphate, acetate, TRIS and HEPES and/or extracting is performed with an extraction medium wherein the weight of the extraction medium is at least 20 times, preferably 100 times the weight of the natural source of allergens.

3. The method of any one of claims 1 to 2 wherein the purification comprises one or more of an ion exchange chromatography step, a gel filtration or size exclusion chromatography step, a precipitation step, a hydrophobic interaction chromatography step, a pseudo affinity or affinity chromatography step or a diafiltration step and/or wherein at least one purification step is performed with a solution comprising a tenside and/or denaturating agent.

4. The method of claim 3 wherein denaturation is performed with mixtures of chaotropic agents and reducing agents selected from the group urea, guanidinium chloride, dithiotreitol, thioglycerol, β-mercaptoethanol and mixtures thereof, preferably wherein the concentration of urea is more than 4 M, preferably more than 5 M and/or the concentration of guanidinium chloride is above 3 M, preferably above 4 M.

5. The method of claims 1 to 4 wherein hydrolysing is performed with an enzyme, preferably pepsin, trypsin or chymotrypsin and/or wherein hydrolysing is performed in the presence of a chaotropic agent, preferably selected from urea and guanidinium chloride.

6. The method of any one of claims 1 to 5 wherein the removal of the peptides is performed by size exclusion chromatography and/or by ultrafiltration, preferably wherein the size exclusion chromatography step is performed in the presence of a chaotropic agent, preferably selected among urea, guanidinium chloride, ethylene glycol, isopropanol and mixtures thereof.

7. A purified allergen hydrolysate obtainable by the method of any one of claims 1 to 6.

8. Use of the allergen hydrolysate of claim 7 for the preparation of a pharmaceutical composition for inducing tolerance, wherein induction of tolerance is used to cure or prevent allergic reactions.

9. Use of the allergen hydrolysate of claim 7 for the preparation of a food composition for inducing tolerance, wherein induction of tolerance is used to prevent allergic reactions.

10. A pharmaceutical composition comprising an allergen hydrolysate of claim 7.

11. The pharmaceutical composition of claim 10 comprising additionally at least one substance selected from the group of nucleoside triphosphates, nucleoside diphosphates, nucleoside monophosphates, nucleic acids, peptide nucleic acids, nucleosides or analogs thereof, immunosuppressive cytokines, compounds inducing expression of immunoproteasomes, 1,25-dihydroxyvitamin D3 or analogs thereof, lipopolysaccharides, endotoxins, heat shock proteins, thioredoxin with either NADPH or NADP-thioredoxin reductase, dithiothreitol, adrenergic receptor agonists such as salbutanol, adrenergic receptor antagonists such as butoxamine, compounds that regulate the expression of the adhesion molecule ICAM-1, N-acetyl-L-cysteine, y-L-glutamyl-L-cysteinyl-glycine (reduced L-glutathione), alpha-2-macroglobulins, inducers for Foxp3 gene expression, flavonoids, isoflavonoids, pterocarpanoids, stilbenes such as resveratrol, tachykinin receptor antagonists, chymase inhibitors, vaccine adjuvant like CpG or MPL or tolerogenic adjuvant like zymosan, beta-1,3-glucan, regulatory T-cell inducer, a muco-adhesive agent for attaching the particle to the intestinal mucosal lining such as a plant lectin, zinc, zinc salts, polysaccharides, vitamins and bacterial lysates.

12. A pharmaceutical composition of claims 10 or 11 wherein the allergens are selected among pollen allergens, milk allergens, venom allergens, egg allergens, weed allergens, grass allergens, tree allergens, shrub allergens, flower allergens, vegetable allergens, grain allergens, fungi allergens, fruit allergens, berry allergens, nut allergens, seed allergens, bean allergens, fish allergens, shellfish allergens, seafood allergens, meat allergens, spices allergens, insect allergens, mite allergens, mould allergens, animal allergens, pigeon tick allergens, worm allergens, soft coral allergens, animal dander allergens, nematode allergens, allergens of Hevea brasiliensis.

13. A pharmaceutical composition according to anyone of claims 10 to 12 for oral administration, for sublingual drug delivery, for enteric drug delivery.

## Patentansprüche

1. Verfahren zur Herstellung eines gereinigten Allergenhydrolysats, umfassend die Schritte:
a) Extrahieren einer natürlichen Quelle von Antigenen, die allergene Proteine umfasst, zur Bildung eines Extrakts;
b) Reinigen des Extrakts zur Entfernung von Nichtproteinkomponenten zur Bildung eines gereinigten Extrakts;
c) Denaturieren des gereinigten Extrakts zur Bildung eines denaturierten gereinigten Extrakts, wobei die Denaturierung mit Gemischen von chaotropen Mitteln und Reduktionsmitteln durchgeführt wird;
wobei der denaturierte gereinigte Extrakt Proteine umfasst, wobei kein einziges Protein 60 Gew.-% oder mehr aller Proteine ausmacht, gemessen durch SDS-PAGE und anschließende Densitometrie, und alle Proteine wenigstens 60 Gew.-% des Trockengewichts des denaturierten gereinigten Extrakts ausmachen, gemessen durch BCA-Assay;
d) Hydrolysieren des denaturierten gereinigten Extrakts mit einem Enzym unter Bildung eines Allergenhydrolysats;
e) Reinigen des Allergenhydrolysats zur Entfernung von Peptiden mit einem Molekulargewicht von über 10 000 Da und unter 1000 Da, um ein gereinigtes Hydrolysat zu erhalten, wobei 70%, besonders bevorzugt 80%, der Peptide zwischen 10 000 Da und 1000 Da liegen.

2. Verfahren gemäß Anspruch 1, wobei das Extrahieren in einer Lösung durchgeführt wird, die kein Salz oder ein aus Carbonat, Hydrogencarbonat, Phosphat, Acetat, TRIS und HEPES ausgewähltes Salz umfasst, und/oder das Extrahieren mit einem Extraktionsmedium durchgeführt wird, wobei das Gewicht des Extraktionsmediums wenigstens das 20-fache, vorzugsweise das 100-fache, des Gewichts der natürlichen Quelle der Allergene beträgt.

3. Verfahren gemäß einem der Ansprüche 1 bis 2, wobei die Reinigung eines oder mehrere aus einem Ionenaustausch-Chromatographieschritt, einem Gelfiltrations- oder Größenausschluss-Chromatographieschritt, einem Fällungsschritt, einem Hydrophobe-Wechselwirkung-Chromatographieschritt, einem Pseudoaffinitäts- oder Affinitätschromatographieschritt oder einem Diafiltrationsschritt umfasst und/oder wobei wenigstens ein Reinigungsschritt mit einer Lösung, die ein Tensid und/oder ein Denaturierungsmittel umfasst, durchgeführt wird.

4. Verfahren gemäß Anspruch 3, wobei die Denaturierung mit Gemischen von chaotropen Mitteln und Reduktionsmitteln, die aus der Gruppe Harnstoff, Guanidiniumchlorid, Dithiothreitol, Thioglycerin, β-Mercaptoethanol und Gemischen davon ausgewählt sind, durchgeführt wird, wobei vorzugsweise die Konzentration von Harnstoff mehr als 4 M, vorzugsweise mehr als 5 M, beträgt und/oder die Konzentration von Guanidiniumchlorid über 3 M, vorzugsweise über 4 M, liegt.

5. Verfahren gemäß Anspruch 1 bis 4, wobei das Hydrolysieren mit einem Enzym, vorzugsweise Pepsin, Trypsin oder Chymotrypsin, durchgeführt wird und/oder wobei das Hydrolysieren in Gegenwart eines chaotropen Mittels, das vorzugsweise aus Harnstoff und Guanidiniumchlorid ausgewählt ist, durchgeführt wird.

6. Verfahren gemäß einem der Ansprüche 1 bis 5, wobei die Entfernung der Peptide durch Größenausschlusschromatographie und/oder durch Ultrafiltration durchgeführt wird, wobei vorzugsweise der Größenausschlusschromatographieschritt in Gegenwart eines chaotropen Mittels, das vorzugsweise aus Harnstoff, Guanidiniumchlorid, Ethylenglycol, Isopropanol und Gemischen davon ausgewählt ist, durchgeführt wird.

7. Gereinigtes Allergenhydrolysat, erhältlich nach dem Verfahren gemäß einem der Ansprüche 1 bis 6.

8. Verwendung des Allergenhydrolysats gemäß Anspruch 7 für die Herstellung einer pharmazeutischen Zusammensetzung zum Induzieren von Toleranz, wobei die Induktion der Toleranz verwendet wird, um allergische Reaktionen zu heilen oder zu verhindern.

9. Verwendung des Allergenhydrolysats gemäß Anspruch 7 für die Herstellung einer Lebensmittelzusammensetzung zum Induzieren von Toleranz, wobei die Induktion der Toleranz verwendet wird, um allergische Reaktionen zu verhindern.

10. Pharmazeutische Zusammensetzung, die ein Allergenhydrolysat gemäß Anspruch 7 umfasst.

11. Pharmazeutische Zusammensetzung gemäß Anspruch 10, die zusätzlich wenigstens eine Substanz umfasst, die aus der Gruppe der Nucleosidtriphosphate, Nucleosiddiphosphate, Nucleosidmonophosphate, Nucleinsäuren, Peptidnucleinsäuren, Nucleoside oder Analoga davon, immunsuppressiven Cytokine, Verbindungen, die die Expression von Immunproteasomen induzieren, 1,25-Dihydroxyvitamin-D3 oder Analoga davon, Lipopolysaccharide, Endotoxine, Hitzeschockproteine, Thioredoxin mit entweder NADPH- oder NADP-Thioredoxin-Reductase, Dithiothreitol, Adrenozeptor-Agonisten, wie Salbutanol, Adrenozeptor-Antagonisten, wie Butoxamin, Verbindungen, die die Expression des Adhäsionsmoleküls ICAM-I regulieren, N-Acetyl-L-cystein, γ-L-Glutamyl-L-cysteinylglycine (reduziertes L-Glutathion), alpha-2-Macroglobuline, Induktoren der Foxp3-Genexpression, Flavonoide, Isoflavonoide, Pterocarpanoide, Stilbene, wie Resveratrol, Tachykinin-Rezeptor-Antagonisten, Chymasehemmer, Impfstoffadjuvans, wie CpG oder MPL, oder tolerogenes Adjuvans, wie Zymosan, beta-1,3-Glucan, regulatorischer T-Zell-Induktor, ein mukoadhäsives Mittel zur Bindung des Partikels an die Darmschleimhaut, wie ein Pflanzenlektin, Zink, Zinksalze, Polysaccharide, Vitamine und bakteriellen Lysate ausgewählt ist.

12. Pharmazeutische Zusammensetzung gemäß Anspruch 10 oder 11, wobei die Allergene aus Pollenallergenen, Milchallergenen, Tiergiftallergenen, Eiallergenen, Unkrautallergenen, Grasallergenen, Baumallergenen, Strauchallergenen, Blumenallergenen, Gemüseallergenen, Getreideallergenen, Pilzallergenen, Fruchtallergenen, Beerenallergenen, Nussallergenen, Samenallergenen, Bohnenallergenen, Fischallergenen, Schalentierallergenen, Meeresfrüchteallergenen, Fleischallergenen, Gewürzallergenen, Insektenallergenen, Milbenallergenen, Schimmelpilzallergenen, Tierallergenen, Taubenzeckenallergenen, Wurmallergenen, Weichkorallenallergenen, Tierhaarallergenen, Nematodenallergenen, Allergenen von *Hevea brasiliensis* ausgewählt sind.

13. Pharmazeutische Zusammensetzung gemäß einem der Ansprüche 10 bis 12 für die orale Verabreichung, die sublinguale Wirkstoffabgabe, die enterische Wirkstoffabgabe.

## Revendications

1. Procédé de production d'un hydrolysat d'allergène purifié comprenant les étapes de
a) extraction d'une source naturelle d'allergènes comprenant des protéines allergéniques pour former un extrait,
b) purification dudit extrait pour éliminer des composants non-protéines pour former un extrait purifié,
c) dénaturation dudit extrait purifié pour former un extrait dénaturé purifié, la dénaturation étant réalisée avec des mélanges d'agents chaotropiques et d'agents réducteurs,
ledit extrait dénaturé purifié comprenant des protéines, dans lequel aucune protéine unique n'est de 60 % ou plus (p/p) de toutes les protéines mesurées par SDS-PAGE suivi par une densitométrie et toutes les protéines représentent au moins 60 % (p/p) du poids sec de l'extrait dénaturé purifié mesuré par un dosage BCA,
d) hydrolyse dudit extrait dénaturé purifié avec une enzyme pour former un hydrolysat d'allergène,
e) purification dudit hydrolysat d'allergène pour éliminer les peptides ayant une masse moléculaire au-dessus de 10 000 Da et en dessous de 1 000 Da afin d'obtenir un hydrolysat purifié où 70 %, de manière davantage préférée 80 % des peptides sont compris entre 10 000 Da et 1 000 Da.

2. Procédé selon la revendication 1, dans lequel l'extraction est réalisée en une solution ne comprenant aucun sel ou un sel choisi parmi le carbonate, le bicarbonate, le phosphate, l'acétate, TRIS et HEPES et/ou l'extraction est réalisée avec un milieu d'extraction dans lequel le poids du milieu d'extraction est au moins 20 fois, de préférence 100 fois le poids de la source naturelle d'allergènes.

3. Procédé selon l'une quelconque des revendications 1 à 2, dans lequel la purification comprend une ou plusieurs parmi une étape de chromatographie par échange ionique, une étape de chromatographie par filtration sur gel ou d'exclusion-diffusion, une étape de précipitation, une étape de chromatographie par interaction hydrophobe, une étape de chromatographie de pseudo-affinité ou d'affinité ou une étape de diafiltration et/ou dans lequel au moins une étape de purification est réalisée avec une solution comprenant un surfactant et/ou un agent de dénaturation.

4. Procédé selon la revendication 3, dans lequel la dénaturation est réalisée avec des mélanges d'agents chaotropiques et d'agents réducteurs choisis dans le groupe de l'urée, du chlorure de guanidinium, du dithiotréitol, du thioglycérol, du β-mercaptoéthanol et de leurs mélanges, de préférence dans lequel la concentration en urée est de plus de 4 M, de préférence de plus de 5 M et/ou la concentration en chlorure de guanidinium est au-dessus de 3 M, de préférence au-dessus de 4 M.

5. Procédé selon les revendications 1 à 4, dans lequel l'hydrolyse est réalisée avec une enzyme, de préférence la pepsine, la trypsine ou la chymotrypsine et/ou dans lequel l'hydrolyse est réalisée en présence d'un agent chaotropique, de préférence choisi parmi l'urée et le chlorure de guanidinium.

6. Procédé selon l'une quelconque des revendications 1 à 5, dans lequel l'élimination des peptides est réalisée par chromatographie d'exclusion-diffusion et/ou par ultrafiltration, de préférence dans lequel l'étape de chromatographie d'exclusion-diffusion est réalisée en présence d'un agent chaotropique, de préférence choisi parmi l'urée, le chlorure de guanidinium, l'éthylène glycol, l'isopropanol et leurs mélanges.

7. Hydrolysat d'allergène purifié pouvant être obtenu par le procédé de l'une quelconque des revendications 1 à 6.

8. Utilisation de l'hydrolysat d'allergène de la revendication 7 pour la préparation d'une composition pharmaceutique destinée à induire une tolérance, dans laquelle l'induction de la tolérance est utilisée pour guérir ou prévenir des réactions allergiques.

9. Utilisation de l'hydrolysat d'allergène de la revendication 7 pour la préparation d'une composition alimentaire destinée à induire une tolérance, dans laquelle l'induction de la tolérance est utilisée pour prévenir des réactions allergiques.

10. Composition pharmaceutique comprenant un hydrolysat d'allergène de la revendication 7.

11. Composition pharmaceutique selon la revendication 10, comprenant de surcroît au moins une substance choisie dans le groupe des triphosphates nucléosidiques, des diphosphates nucléosidiques, des monophosphates nucléosidiques, des acides nucléiques, des acides nucléiques peptidiques, des nucléosides ou de leurs analogues, des cytokines immunosuppressives, des composés induisant une expression d'immunoprotéasomes, la 1,25-dihydroxyvitamine D3 ou ses analogues, des lipopolysaccharides, des endotoxines, des protéines de choc thermique, de la thiorédoxine avec soit NADPH soit NADP-thiorédoxine réductase, du dithiothréitol, des agonistes de récepteur adrénergique tels que le salbutanol, des antagonistes de réception adrénergique tels que la butoxamine, des composés qui régulent l'expression de la molécule d'adhérence ICAM-1, la N-acétyl-L-cystéine, l'y-L-glutamyl-L-cystéinyl-glycine (L-glutathion réduit), des alpha-2-macroglobulines, des inducteurs de l'expression du gène Foxp3, des flavonoïdes, des isoflavonoïdes, des ptérocarpanoïdes, des stilbènes tels que la resvératrol, des antagonistes de récepteur de tachykinine, des inhibiteurs de chymase, de l'adjuvant de vaccin comme au CpG ou MPL ou de l'adjuvant tolérogénique comme Zymosan, le bêta-1,3-glucan, l'inducteur de cellules T régulatrices, un agent muco-adhésif pour attacher la particule à la doublure muqueuse intestinale tel qu'une lectine végétale, le zinc, les sels de zinc, les polysaccharides, les vitamines et les lysats bactériens.

12. Composition pharmaceutique selon la revendication 10 ou 11, dans lequel les allergènes sont choisis parmi les allergènes de pollen, les allergènes de lait, les allergènes de venin, les allergènes de l'oeuf, les allergènes de mauvaise herbe, les allergènes de l'herbe, les allergènes d'arbre, les allergènes d'arbuste, les allergènes de fleur, les allergènes de légume, les allergènes de céréale, les allergènes de champignon, les allergènes de fruit, les allergènes de baie, les allergènes de fruit à coque, les allergènes de graine, les allergènes de haricot, les allergènes de poisson, les allergènes de mollusque et crustacé, les allergènes de poisson et fruit de mer, les allergènes de viande, les allergènes d'épice, les allergènes d'insecte, les allergènes d'acarien, les allergènes de moisissure, les allergènes d'animal, les allergènes de tique du pigeon, les allergènes de ver, les allergènes de main de mer, les allergènes de squame animale, les allergènes de nématode, les allergènes d'*Hevea brasiliensis.*

13. Composition pharmaceutique selon l'une quelconque des revendications 10 à 12, pour une administration orale, pour une délivrance de médicament sublinguale, pour une délivrance de médicament gastro-résistant.
